(19) Europäisches Patentamt
European Patent Office
Office européen des brevets

(11) Numéro de publication: **0 237 454 B1**

# (12) FASCICULE DE BREVET EUROPEEN

(45) Date de publication de fascicule du brevet: **08.05.91**

(51) Int. Cl.5: **G10K 11/16**, A61F 11/06

(21) Numéro de dépôt: **87430008.0**

(22) Date de dépôt: **06.03.87**

## (54) Procédés et dispositifs pour atténuer les bruits d'origine externe parvenant au tympan et améliorer l'intelligibilité des communications électro-acoustiques.

(30) Priorité: **07.03.86 FR 8603394**

(43) Date de publication de la demande: **16.09.87 Bulletin 87/38**

(45) Mention de la délivrance du brevet: **08.05.91 Bulletin 91/19**

(84) Etats contractants désignés: **AT BE CH DE ES FR GB GR IT LI LU NL SE**

(56) Documents cités:
**DE-A- 2 925 134**
**GB-A- 2 160 070**
**US-A- 4 494 074**

(73) Titulaire: **Etablissement Public dit: CENTRE NATIONAL DE LA RECHERCHE SCIENTIFIQUE (CNRS)**
**15, Quai Anatole France**
**F-75007 Paris(FR)**

(72) Inventeur: **Carme, Christian**
**22, Boulevard Rey**
**F-13009 Marseille(FR)**
Inventeur: **Roure, Alain**
**Villa 6 Les Jardins de Montbrun**
**F-13011 Marseille(FR)**

(74) Mandataire: **Azais, Henri et al**
**c/o CABINET BEAU DE LOMENIE**
**"Prado-Mermoz" 232, Avenue du Prado**
**F-13008 Marseille(FR)**

**EP 0 237 454 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

Rank Xerox (UK) Business Services

## Description

La présente invention a pour objet des procédés et des dispositifs pour atténuer les bruits d'origine externe parvenant au tympan et améliorer l'intelligibilité des communications électro-acoustiques.

Le secteur technique de l'invention est celui de la construction des protections acoustiques de l'oreille.

On connaît des moyens d'insonorisation passifs tels que les casques ou coquilles que l'on place sur les oreilles pour protéger les personnes appelées à séjourner dans une ambiance très bruyante. De tels moyens sont utilisés par exemple par les ouvriers travaillant dans certains ateliers, par les conducteurs de véhicules très bruyants (avions, chars....), par le personnel au sol des aéroports etc....

Ces casques comportent généralement une coquille en un matériau absorbant qui enveloppe l'oreille.

Dans ce type d'isolation acoustique, les ondes sonores aériennes incidentes sont atténuées par réflexion et par absorption dans la masse du matériau qui fait fonction d'écran passif.

L'expérience montre que les moyens d'insonorisation passifs sont peu efficaces pour les sons graves, surtout dans la plage des fréquences inférieures à 500 Hz. En effet, pour être efficaces à de telles fréquences, ces casques exigeraient des densités ou des épaisseurs de matériau prohibitives.

On connaît des casques d'isolation acoustique qui comportent, en outre, un haut-parleur ou un transducteur électro-acoustique incorporé à l'intérieur de chaque coquille qui permet à l'usager d'entendre des messages qui lui sont transmis par voie électro-acoustique.

Le problème à résoudre est d'améliorer l'efficacité des casques et autres moyens d'insonorisation passifs en adjoignant à ceux-ci, un dispositif qui permet d'améliorer l'atténuation des sons d'origine externe dans la bande des fréquences où le dispositif passif est peu efficace.

On connaît par ailleurs, des dispositifs dits atténuateurs acoustiques actifs qui permettent d'atténuer certains sons en les faisant interférer avec d'autres sons que l'on crée en opposition de phase avec les sons à atténuer.

Les premières tentatives qui se situent vers 1953 - 1956 ont été décrites par OLSON et MAY.

L'atténuateur acoustique actif proposé par ces auteurs comporte un microphone relié à un haut-parleur par un amplificateur électronique, de sorte que le haut-parleur produit dans la cavité une pression antagoniste de celle de l'onde incidente captée par le microphone. Dans le cas où l'onde incidente est un bruit aléatoire, l'atténuation obtenue par ce procédé n'est pas très bonne et, de plus, ce procédé entraîne des instabilités dues à des résonances sur certaines fréquences (effet LARSEN).

Des travaux inspirés de ceux d'OLSON ont été publiés en 1955-1956 par HAWLEY et SIMSHAUER. Pour éviter les résonances dues à la contre-réaction acoustique, HAWLEY propose de réaliser des casques d'insonorisation qui atténuent uniquement certains bruits gênants qui sont soit des sons harmoniques purs, soit des bruits à bande très étroite. De tels casques ne peuvent atténuer que des bruits déterminés à l'avance et ils ne permettent pas d'atténuer des bruits se situant dans une plage de fréquences étendue.

CHAPLIN et SMITH dans INTER NOISE 1983, pages 399 et 403 décrivent un dispositif anti-bruit qui permet d'atténuer des sons harmoniques en les faisant interférer avec un son synchrone et en opposition de phase qui est engendré par un synthétiseur piloté par une électronique numérique. Ces dispositifs qui utilisent un son synchorne du son à atténuer, ne peuvent servir qu'à atténuer un bruit composé d'une fréquence pure et de ses harmoniques. La variation en fréquence du son doit être lente pour que le traitement numérique puisse corriger la fréquence du signal de contre-réaction. La nécessité d'avoir un signal de synchronisation impose de réaliser un anti-bruit pour chaque source de bruit. L'électronique numérique est complexe et lourde à mettre en oeuvre. Ce procédé n'est donc pas utilisable pour améliorer l'efficacité de moyens d'insonorisation passifs destinés à être utilisés dans des lieux très bruyants dans des champs sonores comportant essentiellement des bruits aléatoires.

Le brevet FR. 75/34.025 (A.N.V.A.R.)(FR-A-2329929) décrit des dispositifs absorbeurs acoustiques actifs qui permettent d'atténuer des ondes acoustiques planes se propageant le long d'un conduit.

Le brevet FR. 83/13.502 (FR-A-2550903) décrit un dispositif de régulation d'une chaîne électro-acoustique selon lequel on incorpore entre l'organe de lecture et le haut-parleur, un filtre ayant une fonction de transfert inverse de la fonction de transfert de l'ensemble constitué par les enceintes et par le local d'écoute.

Le filtre utilisé est un filtre numérique programmable non récursif, du type convolueur muni d'un échantillonneur d'entrée.

Ce brevet décrit également des moyens pour atténuer les bruits qui se propagent le long d'un guide, lesquels moyens comportent un microphone relié à plusieurs haut-parleurs par une chaîne électronique comportant un filtre du type filtre numérique convolueur, qui fait subir aux signaux électriques le filtrage adéquat pour obtenir la minimisation du bruit.

Les moyens décrits dans ces documents antérieurs nécessitent des adaptations importantes pour

pouvoir être utilisés en combinaison avec des moyens d'insonorisation passifs portatifs, du type casques ou bouchons d'oreille.

Le dernier document antérieur décrit des atténuateurs acoustiques actifs comportant un filtre numérique qui réalise une fonction de transfert complexe inverse de la fonction de transfert d'une chaîne électro-acoustique. Une telle solution comportant un filtre numérique exige l'utilisation en temps réel d'une unité de calcul et constitue une solution onéreuse qui est difficilement utilisable avec des moyens d'insonorisation passifs déambulatoires.

Le brevet U.S-A-4.494.074 (BOSE) décrit des écouteurs comportant un microphone et un transducteur électro-acoustique reliés par une boucle de contre-réaction comportant un préamplificateur, un additionneur de signaux, des circuits de compensation comportant des filtres et un amplificateur qui excite le transducteur.

Le brevet G.B-A-2.160.070 (PLESSEY) décrit des atténuateurs de bruits actifs associés à un écouteur. Les atténuateurs comportent un microphone relié à un transducteur électro-acoustique par une boucle de contre-réaction comportant un amplificateur inverseur.

Le brevet DE. A. 2.925.134 (SENNHEISER ELECTRONIC K.G.) décrit des dispositifs de protection active contre les bruits externes qui comportent un microphone relié à un transducteur émetteur electro-acoustique par une boucle de contre-réaction représentée schématiquement qui comporte un amplificateur et qui peut comporter un filtre réglable passe-haut ou passe-bas.

Certains de ces documents antérieurs mentionnent la présence d'un filtre dans la boucle de contre-réaction, mais aucun ne précise comment on choisit ledit filtre et la fonction de transfert de celui-ci pour obtenir la meilleure atténuation possible.

Un objectif de la présente invention est de procurer des moyens d'atténuation actifs associés à des moyens qui délimitent une cavité autour de l'oreille, qui comportent un filtre dont la fonction de transfert est déterminée à partir de la fonction de transfert en boucle ouverte de ladite cavité pour obtenir une bonne atténuation active dans une large plage de fréquences,englobant notamment les sons graves pour lesquels les dispositifs d'insonorisation passifs sont peu efficaces.

Les procédés selon l'invention pour atténuer les bruits d'origine externe parvenant au tympan sont du type dans lequel on associe à chaque oreille un moyen d'insonorisation passif qui délimite avec celle-ci une cavité et on dispose à l'intérieur de ladite cavité un transducteur électro-acoustique et un microphone qui sont reliés l'un à l'autre par une boucle de contre-réaction comportant un amplificateur à gain constant et un filtre qui constituent un insonorisateur actif.

L'objectif de l'invention est atteint au moyen d'un procédé selon lequel la fonction de transfert $C(\omega)$ du filtre est une fonction polynomiale complexe et on mesure d'abord la fonction de transfert en boucle ouverte $H(\omega)$ de l'ensemble formé par le transducteur, le microphone et la cavité délimitée par ledit moyen d'insonorisation passif et l'oreille puis on calcule les coefficients de ladite fonction polynomiale $C(\omega)$ pour que le produit du gain constant dudit amplificateur par le module de ladite fonction de transfert en boucle ouverte $|H(\omega)|$ et par le module de la fonction de transfert dudit filtre $|C(\omega)|$ soit beaucoup plus grand que 1 dans la plage de fréquences où ledit moyen d'insonorisation passif est peu efficace tout en conservant la stabilité du système de contre-réaction.

Un dispositif selon l'invention pour atténuer les sons d'origine externe comporte des moyens d'insonorisation passifs qui délimitent une cavité avec chaque oreille et comporte, en outre, un transducteur électro-acoustique et un microphone qui sont disposés à l'intérieur de ladite cavité et qui sont reliés l'un à l'autre par une boucle de contre-réaction comportant un amplificateur à gain constant et un filtre avec lequel ils constituent un atténuateur de sons actif.

L'objectif de l'invention est atteint au moyen d'un dispositif dans lequel la fonction de transfert $C(\omega)$ du filtre est une fonction polynomiale complexe telle que le produit du module $|C\omega|$ par le module $|H(\omega)|$ de la fonction de transfert en boucle ouverte et par le gain K de l'amplificateur soit supérieur à un dans toute la plage des fréquences à atténuer.

L'invention a pour résultat des moyens permettant d'améliorer l'efficacité des casques et autres moyens d'insonorisation passifs analogues en leur associant un atténuateur de sons actif comportant, dans la boucle de contre-réaction, un filtre qui permet d'optimiser l'efficacité d'un moyen d'insonorisation passif dans une large plage de fréquences.

On sait que les moyens d'insonorisation passifs atténuent mal les sons graves, c'est-à-dire les sons ayant une fréquence inférieure à 500 Hz qui sont très présents dans certains bruits, par exemple dans les bruits de moteurs de véhicules.

Les dispositifs selon l'invention permettent d'améliorer, entre autres, l'atténuation des sons grâce à une boucle de contreréaction comportant un amplificateur à gain constant et un filtre qui est, de préférence, un filtre analogique de type polynomial dont les composants sont calculés pour que le rapport entre la

pression acoustique totale dans la cavité, donc au tympan, et la pression acoustique due aux bruits externes ayant traversé le moyen d'insonorisation passif reste faible dans toute la plage des fréquences que l'on désire atténuer.

La réalisation des filtres analogiques polynomieux est une technique bien connue de l'homme de l'art.

Le module de la fonction de transfert globale d'une boucle de contre-réaction selon l'invention est égal au produit du gain constant (K) de l'amplificateur, par le module de la fonction de transfert $|C(\omega)|$ du filtre et par le module de la fonction de transfert $|H(\omega)|$ en boucle ouverte c'est-à-dire mesurée entre l'entrée du transducteur et la sortie du microphone.

On sait mesurer et tracer le module et l'argument de la fonction de transfert en boucle ouverte $H(\omega)$ en injectant un signal de bruit blanc à l'entrée du transducteur et en envoyant simultanément sur un analyseur spectral ledit signal et le signal émis par le microphone.

Connaissant le module et l'argument de la fonction de transfert en boucle ouverte d'un moyen d'atténuation passif équipé d'un transducteur électro-acoustique et d'un microphone ayant une disposition déterminée, il est possible, après calcul, de déterminer les valeurs des composants passifs d'un filtre pour que le module de la fonction de transfert de celui-ci évolue suivant une loi déterminée dans une plage de fréquences que l'on désire atténuer tout en vérifiant le critère de stabilité, de telle sorte qu'il ne se produise aucune résonance due à un "accrochage" ou effet LARSEN.

Les dispositifs selon l'invention permettent d'atténuer aussi bien des bruits continus que des bruits impulsifs, c'est-à-dire des bruits tels que ceux qui sont provoqués par des chocs ou des détonations dont l'amplitude varie très rapidement. Ceci est possible car le traitement en électronique utilisé est en temps réel.

La description suivante se réfère aux dessins annexés qui représentent, sans aucun caractère limitatif, des exemples de réalisation de l'invention.

La figure 1 est une vue d'ensemble schématique d'un dispositif d'insonorisation selon l'invention.

La figure 2 est une coupe d'une oreille équipée d'un dispositif selon l'invention.

La figure 3 est une vue schématique des composants d'un dispositif selon l'invention.

La figure 4 est un diagramme représentant le module du spectre sonore à l'intérieur d'un casque passif déterminé, lorsque le bruit extérieur est un bruit blanc.

La figure 5 est un diagramme qui montre l'atténuation obtenue par un dispositif selon l'invention.

La figure 6 est une vue schématique des composants d'un dispositif selon l'invention destiné à permettre l'audition de messages transmis par voie électro-acoustique.

La figure 7 est une coupe d'une oreille équipée d'un dispositif selon l'invention.

La figure 1 représente schématiquement un dispositif selon l'invention placé sur les oreilles d'un sujet en vue d'atténuer les bruits externes perçus par celui-ci.

Ce dispositif comporte des moyens d'insonorisation passifs qui sont constitués, par exemple, par deux coquilles 1d, 1g qui enveloppent chaque oreille et qui sont reliées entre elles par un arceau 2 pour former un casque. Les coquilles 1d, 1g sont appliquées contre les côtés de la tête avec lesquels elles délimitent chacune une cavité dans laquelle le pavillon de l'oreille est enfermé. Cette cavité qui est fermée est composée de deux cavités, la première correspondant à l'intérieur de la coquille et la deuxième à la cavité auditive limitée par le pavillon, le conduit auditif et le tympan.

Les coquilles 1d, 1g constituent un dispositif d'insonorisation passif qui réfléchit et absorbe une partie des ondes acoustiques et qui atténue les bruits parvenant à l'oreille. Les coquilles 1d, 1g peuvent être remplacées par tout autre dispositif d'insonorisation passif, par exemple par des bouchons placés à l'entrée des conduits auditifs. Dans ce cas la cavité fermée est composée d'une première demi-cavité semi-ouverte, formée par le bouchon d'oreille avec ses transducteurs miniaturisés et d'une deuxième demi-cavité formée par le conduit auditif et le tympan.

L'expérience montre que les dispositifs d'insonorisation passifs n'atténuent pas bien les sons graves.

La figure 4 est un diagramme qui représente en abscisses la fréquence des sons et on ordonnées le module de la fonction de transfert entre un microphone placé dans un casque passif selon la figure 1 et un bruit blanc extérieur.

On rappelle que la fonction de transfert $H(\omega)$ est une fonction complexe qui exprime le rapport entre la transformée de FOURIER $S(\omega)$ d'un signal sortant d'un dispositif et la transformée de FOURIER $E(\omega)$ du signal appliqué à l'entrée de ce dispositif, donc :

$$H(\omega) = \frac{S(\omega)}{E(\omega)}.$$

La figure 4 indique donc pour chaque fréquence contenue dans le bruit extérieur, le niveau mesuré en

décibels relatifs du bruit à l'intérieur de la cavité délimitée par une des coquilles 1d ou 1g. On voit que pour les fréquences inférieures à 600 Hz, l'atténuation obtenue est moins bonne que pour les fréquences plus élevées.

Le problème à résoudre est d'adjoindre à un dispositif d'insonorisation passif, un moyen d'insonorisation actif permettant d'atténuer principalement les fréquences qui échappent à l'atténuation passive, étant précisé que le moyen actif permet aussi d'améliorer l'atténuation des fréquences plus élevées qui sont déjà atténuées par le moyen passif.

La figure 2 est une coupe d'une oreille sur laquelle on voit le conduit auditif 3, le tympan 16 et la pavillon 4 qui est placé à l'intérieur d'une coquille isolante 1 comportant une garniture 5 en matériau cellulaire, qui est appliquée contre la peau autour du pavillon de l'oreille.

La figure 2 représente un transducteur électro-acoustique 6, par exemple un petit haut-parleur qui est supporté par une cloison 7 fixée sur la coquille isolante 1. La face émissive du haut-parleur 6 est dirigée vers l'oreille. Elle est placée sensiblement en face de l'entrée du conduit auditif 3 et, de préférence, à faible distance de cette entrée, par exemple à une distance de l'ordre de quelques centimètres. Le transducteur 6 est relié par un conducteur électrique à une borne A.

La figure 2 représente, en outre, un microphone 8 qui est disposé dans le conduit auditif 3 ou bien entre la face émissive du haut-parleur 6 et l'entrée du conduit auditif 3 et qui est relié par un conducteur à une borne B.

La figure 1 représente un boîtier 9 qui contient les composants électroniques et les circuits reliant respectivement la borne A à la borne B pour une oreille et la borne A' à la borne B' pour l'autre oreille.

La figure 3 est une représentation schématique de principe sur laquelle on a représenté en coupe, une cavité fermée 10, qui correspond au couplage de la cavité ouverte de la coquille avec la cavité ouverte de l'oreille, dans laquelle sont placés un transducteur électro-acoustique 6 et un microphone 8. Le microphone 8 est relié au transducteur 6 par un circuit électronique comportant un amplificateur 11 à gain constant K et un filtre analogique 12 de type actif, c'est-à-dire un filtre composé d'amplificateurs sélectifs associés à des composants passifs (capacité ou résistances).

On retrouve sur la figure 3 les bornes A et B représentées sur les figures 1 et 2.

Il se produit dans le conduit autidif une interférence entre deux pressions acoustiques antagonistes.

La première pression est due aux bruits qui proviennent de l'extérieur à travers la coquille 1 et qui ont été plus ou moins atténués selon les fréquences. Après transformation de FOURIER, cette pression acoustique peut être représentée par une fonction complexe $Po(\omega)$, où $\omega$ est la pulsation correspondant à chaque fréquence $f(\omega = 2\pi f)$ .

La deuxième pression est celle qui résulte des ondes émises par le transducteur 6 à partir des signaux émis par le microphone 8 amplifiés par l'amplificateur 11 et transformés par le filtre 12. Le circuit partant du microphone, passant par l'amplificateur, le filtre et le haut-parleur et revenant à l'entrée du microphone, constitue une boucle de contre-réaction qui se referme dans le conduit auditif.

On désigne par $P(\omega)$ la fonction complexe représentant la transformée de FOURIER de la pression résultante.

On désigne par K le gain constant de l'amplificateur.

On désigne par $H(\omega)$ la fonction de transfert en boucle ouverte entre les points A et B.

On désigne par $C(\omega)$ la fonction de transfert du filtre 12.

Considérant qu'on est en présence d'une boucle de contreréaction fermée, on peut écrire, en utilisant les transformées de FOURIER, que la pression totale $P(\omega)$ est égale à la somme de la pression incidente $Po(\omega)$ et de la pression due à la contre-réaction qui est égale à : $K.H(\omega).C(\omega).P(\omega)$ .

On aboutit donc aux équations :

$$(1) \qquad P(\omega) = Po(\omega) + K.H(\omega).C(\omega)\ P(\omega)$$

d'où

$$(2) \qquad \frac{P(\omega)}{Po(\omega)} = \frac{1}{1-KH(\omega).C(\omega)}.$$

Cette équation montre qu'on peut atténuer dans une plage de fréquences déterminée la pression acoustique $P(\omega)$ parvenant à l'oreille si on peut arriver à réduite dans toute cette plage le rapport $\frac{P}{Po}$ c'est-à-dire à obtenir une valeur du produit complexe $K.H(\omega).C(\omega)$ très supérieure à 1, tout en évitant les

phénomènes de résonance sur certaines fréquences. En effet, il ne servirait à rien d'atténuer les bruits dans une plage de fréquences si par ailleurs on créait des bruits parasites encore plus gênants par effet LARSEN.

L'examen de l'équation (2) montre que si l'on pouvait réaliser un filtre ayant une fonction de transfert C-($\omega$) = $H^{-1}(\omega)$ dans toute une plage de fréquences donnée, il suffirait de choisir un amplificateur ayant un gain K très élevé pour obtenir une très bonne atténuation des bruits dans cette plage de fréquences.

Il est possible d'approcher une fonction de transfert inverse de la fonction de transfert en boucle ouverte en utilisant des filtres numériques associés à une unité de calcul, mais c'est une solution encombrante, onéreuse et ne permettant pas de travailler en temps réel comme le nécessite le système de contre-réaction.

Le filtre 12 utilisé dans un dispositif selon les figures 1 à 3 est un filtre analogique d'encombrement et de coût réduits, qui ne permet pas de réaliser une fonction de transfert inverse de la fonction de transfert en boucle ouverte.

La fonction de transfert en boucle ouverte H($\omega$) peut être mesurée en supprimant le coffret 9, en envoyant sur la borne A un signal électrique d'entrée qui correspond à un bruit blanc et en recueillant en B le signal électrique de sortie émis par le microphone 8.

Il suffit d'envoyer simultanément les deux signaux électriques d'entrée et de sortie sur un analyseur spectral qui est programmé pour effectuer la conversion analogique-numérique des deux signaux et pour calculer la fonction de transfert en boucle ouverte H($\omega$) correspondant à des fréquences discrètes.

L'analyseur spectral comporte un écran sur lequel il affiche, d'une part, les variations du module de la fonction de transfert et, d'autre part, la phase en fonction de la fréquence.

L'analyse spectrale montre que la fonction de transfert en boucle ouverte, c'est-à-dire le rapport entre les transformées de FOURIER du signal de sortie au point B et du signal d'entrée au point A dépend beaucoup de la forme et du volume de la cavité 10 et également des positions respectives et par rapport à la cavité du transducteur 6 et du microphone 8.

Les études réàlisées en laboratoire ont montré que l'on pouvait réduire les variations du module de la fonction de transfert en boucle ouverte et les déphasages.

En effet, plutôt que d'utiliser la fonction de transfert en boucle ouverte H($\omega$) telle quelle, il est possible d'optimiser cette fonction de transfert avant le traitement électronique. Optimiser une fonction de transfert existante dans une boucle de contre-réaction, permet d'obtenir une atténuation acoustique active plus importante et ce, sur une plage de fréquences plus large. L'optimisation de la fonction de transfert en boucle ouverte revient à réaliser une "pseudo-linéarisation" de celle-ci, de telle sorte que le module et la phase de H($\omega$)soient constants et que le déphasage soit faible dans la plage de fréquences à atténuer selon le principe de l'invention. Ainsi, plus la fonction de transfert H($\omega$) est "linéaire" et plus le traitement électronique pour la contre-réaction est simplifié.

Le procédé proposé pour optimiser la fonction de transfert en boucle ouverte H($\omega$) consiste en une succession d'étapes. Tout d'abord (cf.figures 2&3) on divise la cavité 10 en deux cavités à l'aide d'une cloison 7 (dans le cas d'un haut-parleur peu ou pas bafflé) où la "cavité avant" correspond à l'ensemble constitué par les éléments 3, 4, 5, 6, 7, 16 et la "cavité arrière" correspond à l'ensemble formé par les éléments 1, 6, 7, puis on place le microphone 8 dans la "cavité avant" décrite précédemment soit en avant du haut-parleur et à proximité de l'entrée du conduit auditif, soit à l'intérieur de celui-ci, puis on place le microphone à faible distance du haut-parleur afin de réduire le déphasage et on réduit au maximum le volume de la cavité 10 afin d'éviter les effets de résonance et d'antirésonance.

La fonction de transfert en boucle ouverte H($\omega$) pouvant être relativement différente selon les formes géométriques de l'enceinte 10 et les positions du transducteur 6 et du microphone 8, dans la pratique, pour améliorer, par un système actif, l'insonorisation d'une coquille passive 1, de forme et de nature déterminées, il faut commencer par déterminer et fixer la position du transducteur 6 et du microphone 8 à l'intérieur de la coquille 1, pour mesurer ensuite au moyen d'un analyseur spectral, la fonction de transfert en boucle ouverte H($\omega$) de cet ensemble d'éléments qui est posé sur l'oreille.

La figure 7 représente une coupe schématique d'un autre mode de réalisation d'un dispositif selonl'invention. Les parties homologues à celles de la figure 2 sont représentées par les mêmes repères.

Dans le mode de réalisation selon la figure 7, on intercale entre la cloison 7 et le pavillon d'oreille 4 et à l'intérieur de la garniture 5, une pièce annulaire 15 qui délimite une cavité intermédiaire 15a. Avantageusement, cette pièce 15 sert de support du microphone 8 qui peut être disposé dans un évidement de la pièce 15 comme le montre la figure 7 ou bien juxtaposé à ladite pièce 15.

Selon les plages de fréquences à atténuer, on peut optimiser la fonction de transfert en boucle ouverte H($\omega$) par un moyen de filtrage acoustique. Si l'on désire atténuer par exemple les basses fréquences, une pièce annulaire 15 fixée contre la cloison 7 produit un filtrage acoustique par un effet de cavité. Dans

l'exemple selon la figure 7, les rapports des diamètres, des épaisseurs, de l'ouverture de l'écran (baffle) 7, de la pièce annulaire 15 et du conduit auditif 3, définissent un filtre acoustique passe-bas. On peut donc ainsi modeler la fonction de transfert H(ω) en calculant et en réalisant un préfiltrage acoustique avant le traitement électronique.

On calcule les dimensions de la pièce intermédiaires 15 et de la cavité intermédiaire 15a qu'elle délimite, de telle sorte que le rapport entre les dimensions de ladite cavité intermédiaire et des cavités avant et arrière séparées par la cloison 7, conduise à un filtrage acoustique dont la bande passante correspond à la plage de fréquences à atténuer.

De préférence, la face active du microphone est orientée vers la face émissive du haut-parleur afin d'optimiser la fonction de transfert en boucle ouverte H(ω).

Une fois la fonction de transfert H(ω) établie, on calcule un filtre 12 pour qu'il réalise une fonction de transfert C(ω) telle que le produit du gain constant K de l'amplificateur 11 par le module de la fonction de transfert en boucle ouverte H(ω) et par le module de la fonction de transfert C(ω) du filtre 12 soit beaucoup plus grand que 1 à l'intérieur de la plage de fréquences où l'on désire améliorer l'atténuation du bruit externe. Cette condition n'est pas suffisante. Il faut, de plus, vérifier le critère de stabilité afin d'éviter des phénomènes "d'accrochage acoustique" qui conduiraient à la production de bruits par suite de résonances sur certaines fréquences (effet LARSEN).

Pour éviter l'effet LARSEN dû à des résonances, il faut que le critère de stabilité de NYQUIST qui est bien connu des électriciens soit rempli.

On rappelle brièvement que le critère de NYQUIST consiste à vérifier sur un graphique dit de NYQUIST que la fonction de transfert totale F(ω) de l'ensemble des éléments d'un système ne coupe pas l'axe des parties réelles en un point d'abscisse supérieur à 1 pour toutes les fréquences du spectre d'audition.

Le tracé du graphique de NYQUIST consiste à porter en abscisses la partie réelle de la fonction de transfert et en ordonnées, la partie imaginaire.

Soit F(ω) la fonction de transfert totale du système en boucle ouverte.

Soit |F(ω)| le module et $\phi(\omega)$ la phase de cette fonction.

Soit $\Delta\phi$ la marge de stabilité en phase et $\Delta\rho$ la marge de stabilité en module.

La marge de stabilité en phase $\Delta\phi$ correspond aux variations en radians de la phase de la fonction de transfers F(ω) dues à des retards parasites imprévisibles que la phase peut subir sans que le système devienne instable.

La marge de stabilité en module $\Delta\rho$ correspond aux variations imprévues du module de la fonction de transfert F(ω) que peut subir le système de contre-réaction sans pour autant devenir instable.

On démontre que l'on obtient, au moyen d'un boucle de contre-réaction électro-acoustique, une atténuation acoustique active dans une plage de fréquences ωi, sans engendrer par résonance un système instable si et seulement si :

$$|F(\omega i)| > 1 - \Delta\rho$$

et $$2K\pi + \Delta\phi < \phi(\omega i) < 2K\pi - \Delta\phi \quad \text{avec } K = 0,1,2....$$

si $$|F(\omega i)| \lesssim 1 - \Delta\rho$$

le système ne produit aucune atténuation active et, dans ce cas, le système est stable quelle que soits la valeur de la phase $\phi(\omega)$.

Si ces deux conditions sont remplies, les ondes acoustiques qui n'ont pas été arrêtées par le dispositif d'insonorisation passif 1 et qui parviennent à l'entrée du conduit auditif, interfèrent avec les ondes acoustiques émises par le haut-parleur 6 et cette interférence conduit à une minimisation du rapport $\frac{P}{Po}$ entre le module P de l'onde acoustique résultante et le module Po de l'onde acoustique incidente dans toute la plage de fréquences où le produit K. |H(ω)| . |C(ω)| est très supérieure à 1. De plus, il n'apparaît aucun son parasite dû à l'effet LARSEN.

Dans la pratique, le filtre utilisé 12 est, de préférence, un filtre analogique actif, composé par exemple d'un ou plusieurs filtres en circuits intégrés ayant chacun une fonction de transfert polynomiale de la forme :

$$C(\omega) = \frac{a1(\omega)^2 + a2(\omega) + a3}{b1(\omega)^2 + b2(\omega) + b3}.$$

Ce filtre comporte des résistances et/ou des capacités qui peuvent être connectées sur les bornes du circuit intégré et dont les valeurs peuvent être ajustées pour obtenir des valeurs déterminées des coefficients constants réels a1, a2, a3, b1, b2, b3 de la fonction C(ω).

Les calculs permettant de construire des filtres polynomiaux ainsi que les formes des fonctions de transfert de ces filtres sont bien connus des électroniciens.

En général, les coefficients du dénominateur b1, b2, b3 sont fixés à l'avance et ils déterminent la fréquence de coupure et le coefficient de surtension Q du filtre et on fait varier a1, a2, a3 du numérateur pour déterminer la nature du filtre.

Si a1 et a2 sont égaux à zéro et a3 différent de zéro, on réalise un filtre passe-bas qui est intéressant car il réalise une fonction de transfert ayant un module élevé pour les basses fréquences. Mais un tel filtre produit une rotation de phase passant simultanément à 0° et ± 180° et ce, pour une fréquence inférieure à la fréquence de coupure où le module |C(ω)|est > 1, ce qui conduit à un effet LARSEN, qui se produit dans notre cas à 0° si K est positif.

Si a2 et a3 sont nuls et a1 différent de zéro, on réalise un filtre passe-haut qui n'est pas intéressant car les sons à atténuer en priorité sont des sons graves et médium.

Si a1 et a3 sont nuls et a2 différent de zéro, on obtient un filtre passe-bande qui introduit une rotation de phase, par exemple de ± 90° à ∓ 90° sans passer par 0°, et par ± 180° lorsque le module du filtre,c'est-à-dire |C(ω)| est le plus important, ce qui est intéressant car on ne risque pas d'engendrer un effet LARSEN pour notre système.

Dans la pratique, lorsqu'on désire atténuer principalement les sons graves, on utilise, de préférence, des filtres mixtes qui combinent les effets d'un filtre passe-bande et d'un filtre passe-bas. On peut également associer en parallèle plusieurs circuits polynomiaux de type mixte (combinant passe-haut, passe-bas, passe-bande) ou uniquement des passe-bande.

La figure 5 est un diagramme qui représente en abscisses et en coordonnées logarithmique les fréquences audibles et en ordonnées, le niveau des pressions acoustiques exprimé en décibels. La courbe en traits pleins Po représente le spectre de la pression acoustique Po mesurée dans le cas où l'on utilise seulement un casque d'insonorisation passif qui atténue mal les fréquences inférieures à 1500 Hz. La courbe en traits mixtes P représente le spectre de la pression acoustique mesurée lorsque le même casque est associé à un atténuateur actif selon l'invention. Ici, l'électronique utilisée est un simple filtre analogique actif de type passe-bande.

Les pressions acoustiques ont été mesurées en présence d'un bruit blanc émis par une enceinte acoustique dans la bande comprise entre 20 Hz et 20000 Hz. Les pressions acoustiques sont mesurées à l'aide d'un petit microphone qui est introduit dans le conduit auditif le plus près possible du tympan et elles présentent donc les sons perçus par les oreilles. Les spectres Po et P sont obtenus à l'aide d'un analyseur spectral qui applique aux mesures une transformation de FOURIER.

Le diagramme de la figure 5 représente les résultats moyens mesurés au cours de très nombreux essais. Ce diagramme montre que l'on obtient une atténuation comprise entre 0db et 8db dans une bande de fréquences comprises entre 20 Hz et 55 Hz, une très bonne atténuation comprise entre 0db et 50db dans une bande de fréquences comprises entre 65 Hz et 2000 Hz et une légère atténuation entre 3800 Hz et 20 000 Hz.

Il existe deux bandes de fréquences étroites entre 55 et 65Hz et entre 2000 et 3800 Hz sans atténuation, dans lesquelles il y a même une légère augmentation du niveau du bruit qui reste inférieure à 6 db.

Avantageusement, on place en parallèle dans la boucle de contre-réaction, plusieurs filtres passe-bande, par exemple des filtres d'ordre deux ou trois ayant des bandes passantes juxtaposées qui couvrent la majeure partie de la plate où la rotation de phase du système en boucle ouverte comprenant les filtres vérifie le critère de stabilité de Nyquist.

Ce montage en parallèle de plusieurs filtres présente l'avantage qu'il permet d'additionner les bandes passantes des filtres individuels en introduisant une rotation de phase qui est la moyenne des rotation de phase des filtres individuels et qui reste donc comprise entre - 90° et - 270° pour des filtres d'ordre deux entre - 45° et - 315° pour des filtres d'ordre trois, c'est-à-dire dans des domaines vérifiant le critère de stabilité de Nyquist.

Selon un exemple préférentiel, on utilise un filtre passe-bande avec un filtre passe-haut ayant même fréquence de coupure et même coefficient de surtension.

Ce résultat est obtenu en utilisant un circuit intégré associé à des résistances variables dont le réglage permet d'ajuster les trois coefficients a1, a2, a3 du numérateur de la fonction de transfert polynomiale.

Selon les cas, on peut ainsi obtenir soit un filtre passe-bande prédominant en privilégiant le coefficient a2 par rapport aux deux coefficients a1 et a3, soit un filtre passe-bas prédominant en privilégiant le

coefficient a3.

Le filtre passe-haut contenu dans un tel filtre composé a pour effet de réduire la rotation de phase et de la faire tendre vers zéro pour les hautes fréquences sans modifier sensiblement l'allure du module, ce qui permet d'élargir la bande passante d'atténuation vers les hautes fréquences sans introduire d'instabilité et donc d'augmenter le niveau d'atténuation et la larguer de bande atténuée.

Au lieu d'associer trois filtres ayant même fréquences de coupure et même coefficient de surtension, on peut aussi monter en parallèle plusieurs filtres de type passe-bas, passe-bande et passe-haut ayant des fréquences de coupure différentes, telles que la fréquence de coupure la plus basse soit celle du filtre passe-haut et la plus haute, celle du filtre passe-bas et ayant des coefficient de surtension différents.

Cette association permet d'obtenir des bandes passantes à front plus raide que les précédents et dont la rotation de phase tend vers zéro en dehors de la bande passante, aussi bien dans les fréquences élevées dans dans les basses fréquences.

Tous ces réglages peuvent être faits en faisant d'abord une simulation numérique à partir de la fonction de transfert en boucle ouverte mesurée H(ω) et, au besoin, en ajustant manuellement des résistances variables qui permettent de déterminer la fonction de transfert optima C(ω) du filtre.

Tous ces exemples montrent clairement les possibilité très étendues des procédés selon l'invention qui permettent d'obtenir des atténuateurs de sons actifs très performants qui peuvent être implantés sur des casques d'insonorisation existants ou sur des casques conçus spécialement pour recevoir de tels atténuateurs.

La courbe P1 de la figure 5 représente un exemple de courbe d'atténuation obtenue en associant en parallèle plusieurs filtres analogiques d'ordre deux de type passe-bas, passe-bande et passe-haut, ayant des fréquences de coupure et des coefficients de surtension différents. On voit que l' on obtient une atténuation dans une bande plus large que celle qui est représentée sur la courbe P et que l'on supprime les pics d'augmentation du niveau de bruit.

Très souvent, les casques d'isolation acoustique comportent unhaut-parleur incorporé pour transmettre à l'usager des messages par voie électro-acoustique.

Si l'on équipe un casque selon l'invention qui comporte un atténuateur actif d'un deuxième haut-parleur n'appartenant pas à la boucle de contre-réaction et destiné à transmettre des messages, les sons émis par ce deuxième haut-parleur sont considérés comme des sons venant de l'extérieur donc repris par le microphone et par la boucle de contre-réaction et sont atténués, donc cette solution doit être écartée.

Une autre solution consiste à envoyer les signaux électriques véhiculant le message directement sur le haut-parleur 6 qui fait partie de l'atténuateur actif.

Les équations et l'expérience montrent que ce montage permet d'entendre le message bien qu'il soit modifié par la contreréaction. Mais il existe un meilleur montage.

La figure 6 est un schéma analogue à celui de la figure 3, qui représente ce montage. Les parties homologues à celles de la figure 3 sont représentées par les mêmes références.

Le coffret 9 contient, outre l'amplificateur à gain constant 11 et le filtre 12, un circuit sommateur 13, qui reçoit, d'une part, les signaux émis par le microphone 8 et, d'autre part, des signaux électriques qui proviennent d'un transducteur externe 14 et qui véhiculent un message.

L'expérience montre qu'un tel montage permet non seulement d'atténuer les bruits qui parviennent au tympan sans atténuer le niveau du message mais permet en plus, de s'affranchir de la fonction de transfert du haut-parleur 6 et de celle de la cavité 10 dans la plage de fréquences considérées.

Dans les formules ci-après, K, C(ω), Po(ω), Pt(ω) et H(ω) ont la même signification que dans les formules (1) et (2).

La fonction de transfert en boucle ouverte H(ω) entre les points A et B peut être décomposée en un produit d'une première fonction de transfert H1(ω) entre le point A et l'entrée du microphone et une deuxième fonction de transfert H2 (ω) qui est celle du microphone. En pratique, le module de la fonction de transfert du microphone peut être sensiblement constant dans une large bande de fréquences si le microphone est de bonne qualité.

On désigne par SP(ω) la transformée de FOURIER du signal électrique qui est émis par le transducteur externe 14 et qui véhicule le message.

On peut écrire l'équation suivante :

$$(3) \qquad Pt(\omega) = \frac{K.H1(\omega).C(\omega).Sp(\omega)+Po(\omega)}{1-K.H(\omega).C(\omega)}$$

Si le gain K de l'amplificateur est très grand, on peut écrire :

$$(4) \qquad Pt(\omega) \approx \frac{H1(\omega)}{H(\omega)} \cdot Sp(\omega) + \varepsilon \approx H2(\omega).Sp(\omega) + \varepsilon.$$

Cette équation (4) montre que le montage selon la figure 6 permet à la fois d'atténuer le résidu du bruit d'origine externe grâce à la boucle de contre-réaction mais également d'améliorer l'audition du message qui ne subit plus la fonction de transfert $H1(\omega)$ qui représente la fonction de transfert de l'ensemble cavité et haut-parleur.

Le montage selon la figure 6 permet de réaliser une sorte de filtrage par boucle de contre-réaction d'un message mélangé à un bruit extérieur.

Si l'on considère la réponse du microphone 8 comme idéale, c'est-à-dire $H1(\omega) \approx H(\omega)$, l'équation (4) donne : $Pt(\omega) \cong Sp(\omega) + \epsilon$.

Le montage proposé n diminue pas le niveau du message $Sp(\omega)$ qui se retrouve non seulement intact mais encore affranchi de toute fonction de transfert, ce qui n'est pas le cas si le signal sortant du transducteur 14 était envoyé directement sur le haut-parleur 6.

On voit donc que la solution selon la figure 6 permet d'entendre un message qui est envoyé par voie électro-acoustique et transmis par le même transducteur 6 servant à l'atténuateur actif, après avoir relevé son niveau par rapport à celui des bruits extérieurs et en l'améliorant grâce au fait que l'on supprime la fonction de transfert $H1(\omega)$ qui apparaît inévitablement dans tout autre montage.

Les équations et les mesures expérimentales montrent que toute autre position du sommateur dans la boucle de contre-réaction ne permet pas l'amélioration de l'intelligibilité du message par "filtrage par contre-réaction" et conduit à une altération parfois très importante de l'intelligibilité dudit message transmis. On peut encore améliorer l'intelligibilité du message selon le montage de la figure 6 à condition de centrer la plage des fréquences à atténuer sur le spectre de la parole 300-3000 Hz. En effet, cette plage de fréquences correspond à la zone la plus sensible de l'oreille humaine, ainsi dans le cas d'une atténuation active utilisée surtout pour améliorer l'intelligibilité du message, il n'est plus nécessaire d'atténuer fortement les fréquences graves comme pour le cas d'une protection pure sans communication, mais au contraire, d'améliorer l'atténuation passive à partir de 300 Hz et ce, en élargissant au maximum la plage des fréquences atténuées pour atteindre 2000 voire 3000 Hz, quitte à perdre un peu en efficacité en atténuation pure du bruit.

Les descriptions précédentes s'intéressent surtout aux plages de fréquences basses et médium. Dans ce cas, les équations montrent que la combinaison de l'atténuation acoustique et du "filtrage par contre-réaction" du message transmis améliore les conditions de travail des personnes et l'intelligibilité du message. Toutefois, on vient de voir que si la plage d'atténuation active des fréquences est centrée sur le spectre de la parole 300-3000 Hz, même si l'atténuation n'est pas très importante dans la zone des fréquences aigues, l'intelligibilité du message transmis est encore meilleure et ce, d'autant plus que la plage de fréquences atténuées. s'étend vers les fréquences aigues 2000-3000 Hz.

L'équation (3) montre également que si $Po(\omega)$ est très faible, le phénomène de filtrage par contre-réaction du message est conservée puisque le deuxième membre de l'équation est indépendant de la pression $Po(\omega)$ on vérifie donc encore le résultat de l'équation (4) pour le gain K grand devant 1, c'est-à-dire le message $Sp(\omega)$ se trouve affranchi de toute fonction de transfert. On peut donc utiliser le système d'absorption acoustique active uniquement pour améliorer le message par filtrage par boucle de contre-réaction, même lorsque le bruit extérieur n'est pas gênant.

La description qui précède se réfère à des modes de réalisations préférentiels dans lesquels le filtre 12 est une filtre analogique. Il est précisé que l'on pourrait utiliser également un filtre numérique seul ou associé à une unité de calcul, en combinaison avec un dispositif d'insonorisation passif utilisé à bord d'un véhicule.

Selon une variante de réalisation, un dispositif actif selon l'invention comporte un petit microphone qui est placé dans le conduit auditif et un transducteur miniaturisé dont la face arrière porte un revêtement qui forme un bouchon qui est engagé dans l'entrée du conduit autidif. Dans ce cas, la cavité est réduite au volume délimité par le conduit auditif, le tympan et le bouchon portant le transducteur et la fonction de transfert en boucle ouverte $H(\omega)$ est très linéaire, de sorte que l'on peut obtenir facilement, par filtrage électronique, un bon niveau d'atténuation dans une large bande de fréquences.

## Revendications

1. Procédé pour atténuer les bruits d'origine externe parvenant au tympan, du type dans lequel on

associe à chaque oreille un moyen d'insonorisation passif (1g, 1d) qui délimite avec celle-ci une cavité (10) et on dispose à l'intérieur de ladite cavité un transducteur électro-acoustique (6) et un microphone (8) qui sont reliés l'un à l'autre par une boucle de contre-réaction comportant un amplificateur à gain constant (11) et un filtre (12) qui constituent un insonorisateur actif, caractérisé en ce que la fonction de transfert C(ω) dudit filtre (12) est une fonction polynomiale complexe, en ce que l'on mesure d'abord la fonction de transfert en boucle ouverte H(ω) de l'ensemble formé par le transducteur (6), le microphone (8) et la cavité (10) délimitée par ledit moyen d'insonorisation passif et l'oreille et en ce que l'on calcule en suite les coefficients de ladite fonction polynomiale C(ω) pour que le produit du gain constant (K) dudit amplificateur (11) par le module de ladite fonction de transfert en boucle ouverte |H(ω)| et par le module de la fonction de transfert dudit filtre |C(ω)| soit beaucoup plus grand que 1 dans la plage de fréquences où ledit moyen d'insonorisation passif est peu efficace tout en conservant la stabilité du système de contre-réaction.

2. Procédé selon la revendication 1, caractérisé en ce que pour mesurer ladite fonction de transfert en boucle ouverte H(ω), on débranche ledit amplificateur (11) et ledit filtre (12), on envoie un signal électrique correspondant à un bruit blanc à l'entrée (A) dudit transducteur et on mesure ladite fonction de transfert au moyen d'un analyseur spectral qui reçoit simultanément ledit signal électrique et le signal émis par ledit microphone (8).

3. Procédé selon la revendication 1, caractérisé en ce qu'on modifie ladite fonction de transfert en boucle ouverte H(ω) en divisant ladite cavité (10) par une cloison (7) en deux demi cavités, une demi-cavité avant délimitée par le pavillon de l'oreille (4), le conduit auditif (3), le tympan (16) et ladite cloison (7) et une demi-cavité arrière délimitée par ledit moyen d'insonorisation passif (1) et ladite cloison (7), laquelle cloison (7) porte ledit transducteur (6) et on place ledit microphone (8) dans ladite demicavité avant, le plus près possible de la face émissive dudit transducteur (6).

4. Procédé selon la revendication 3, caractérisé en ce que l'on réduit le plus possible le volume de ladite cavité (10) afin de "linéariser" ladite fonction de transfert en boucle ouverte H(ω).

5. Procédé selon la revendication 3, caractérisé en ce qu'on intercale entre ladite cloison (7) et le pavillon de l'oreille (4), une pièce annulaire (15) qui délimite une cavité intermédiaire (15a) et on calcule la forme et les dimensions de ladite pièce annulaire (15) pour qu'elle réalise un filtrage acoustique qui permet de donner à ladite fonction de transfert en boucle ouverte H(ω), une fonction de filtre passe-bas ou passe-bande selon la plage des fréquences à atténuer.

6. Procédé selon la revendication 1 pour atténuer les bruits d'origine externe par un moyen d'insonorisation actif placé à l'entrée des oreilles tout en permettant d'entendre un message transmis par voie électro-acoustique du type dans lequel on mélange les signaux électriques véhiculant lesdits messages aux signaux émis par ledit microphone et on envoie lesdits signaux mélangés sur ledit transducteur (6) en passant par ledit amplificateur (11), caractérisé en ce que lesdits signaux mélangés passent également à travers ledit filtre (12).

7. Dispositif pour atténuer les bruits d'origine externe parvenant au tympan, du type comportant des moyens d'insonorisation passifs (1d, 1g) qui délimitent une cavité (10) avec chaque oreille et comportant, en outre, un transducteur électro-acoustique (6) et un microphone (8) qui sont disposés à l'intérieur de ladite cavité et qui sont reliés l'un à l'autre par une boucle de contre-réaction comportant un amplificateur à gain constant (11) et un filtre (12) avec lesquels ils constituent un atténuateur de sons actif, caractérisé en ce que la fonction de transfert (Cω) dudit filtre est une fonction polynomiale complexe, en ce que le produit du gain constant (K) dudit amplificateur (11) par le module |C(ω)| de la fonction de transfert dudit filtre et par le module |H(ω)| de la fonction de transfert en boucle ouverte mesurée entre l'entrée (A) dudit transducteur (6) et la sortie (B) dudit microphone (8) est nettemment supérieur à un dans toute la plage des basses fréquences acoustiques qui doivent être atténuées et en ce que la boucle de contre-réaction est stable pour toutes les fréquences audibles.

8. Dispositif selon la revendication 7, caractérisé en ce que ledit filtre (12) comporte un ou plusieurs filtres analogiques de type passe-bande ou passe-bande et passe-bas qui sont montés enparallèle et qui réalisent une fonction de transfert permettant d'éviter des instabilités dans la zone où le module est le plus important.

9. Dispositif selon la revendication 7, caractérisé en ce que ledit filtre comporte plusieurs filtres analogiques de type passe-bas, passe-bande et passe-haut, qui sont montés en parallèle et qui ont même fréquence de coupure et même coefficient de surtension.

10. Dispositif selon la revendication 7, caractérisé en ce que le gain (K) dudit amplificateur (11) est positif et la fonction de transfert C(ω) dudit filtre est déterminée de telle sorte que la phase (ϕ) de ladite fonction de transfert ne passe pas par la valeur zéro dans la bande passante dudit filtre.

11. Dispositif selon la revendication 7, du type dans lequel chacune desdites cavités comporte une cloison transversale (7) qui la divise en deux demi-cavités avant et arrière et qui porte ledit transducteur acoustique et dans lequel ledit microphone (8) est disposé dans ladite demi-cavité avant, caractérisé en ce qu'il comporte, en outre, une pièce annulaire (15) qui est intercalée entre ladite cloison (8) et le pavillon (4) de l'oreille et qui délimite une cavité intermédiaire (15a) et les dimensions de ladite pièce annulaire (15) sont calculées pour que le rapport entre les dimensions de ladite cavité intermédiaire et desdites demi-cavité avant et arrière conduise à un filtrate acoustique dont la bande passante correspond à la plage de fréquences à atténuer.

12. Dispositif selon la revendication 7, caractérisé en ce que ledit microphone (8) est placé dans le conduit auditif (3) et ledit transducteur (6) est un transducteur miniaturisé dont le revêtement de la face arrière forme un bouchon qui est engagé dans l'entrée du conduit auditif (3), de sorte que ladite cavité est réduite au volume délimité par le conduit auditif (3), le tympan (16) et ledit transducteur (6) et que la fonction de transfert en boucle ouverte H(ω) est très linéaire et permet d'obtenir par filtrage électronique, un bon niveau d'atténuation dans une large bande de fréquences.

13. Dispositif selon la revendication 8, caractérisé en ce que ledit filtre comporte, en outre, un ou plusieurs filtres passe-haut montés en parallèle avec lesdits filtres passe-bas et passe-bande.

## Claims

1. Procedure for attenuating sounds of exterior origin arriving at the eardrum, of the type in which each ear is provided with a passive means of soundproofing (1g, 1d) which encloses a cavity (10) around the ear, inside of which cavity there is an electroacoustic transducer (6) and a microphone (8) linked to one another by a feedback loop containing a constant gain amplifier (11) and a filter (12) that make up an active soundproofing system. characterised by the transfer function C(W) of the said filter (12) being a complex polynomial function, in which the open loop transfer function H(w) is firstly measured for the system made up of the transducer (6), microphone (8) and the cavity (10) enclosed by the said passive soundproofing system and the ear and in which the coefficients of the said polynomial function C(w) are calculated so that the product of the constant gain (K) of the amplifier (11) and the modulus of the said open loop transfer function |H(w)| and the modulus of the transfer function of the said filter |C(w)| is much greater than 1 in the range of frequencies over which the said passive soundproofing system is ineffective, while at the same time retaining the stability of the feedback system.

2. Procedure according to claim 1, characterised by the measurement of the said open loop transfer function H(w) being carried out with the said amplifier (11) and the said filter (12) disconnected and an electrical signal corresponding to white noise being sent to the input (A) of the said transducer, the said transfer function is then measured by means of a spectral analyser which simultaneously receives the said electrical signal and the signal emitted by the said microphone (8).

3. Procedure according to claim 1, characterised by the said open loop transfer function H(w) being modified by dividing the said cavity (10) into two half-cavities by a partition (7), a forward half-cavity limited by the ear pinna (4), the auditory passage (3) the eardrum (16) and the said partition (7) and the rear half-cavity limited by the said passive soundproofing system (1) and the said partition (7), the partition (7) supports the said transducer (6) and the said microphone (8) is placed in the said forward half-cavity as close as possible to the emitting face of the said transducer (6).

4. Procedure according to claim 3, characterised by reducing as much as possible the volume of the said cavity (10) in order to make the said open loop transfer function H(w) linear.

5. Procedure according to claim 3, characterised by interposing between the said partition (7) and the ear pinna (4) a ring-shaped component (15) which limits an intermediate cavity (15a) and by designing the shape and form of the said ring-shaped component (15) so that it produces an acoustic filtration enabling it to provide the said open loop transfer function H(w) with the function of a low-pass filter or a pass-band filter according to the frequency range to be attenuated.

6. Procedure according to claim 1, for attenuating sounds of external origin by means of an active soundproofing system placed at the entrance of the ears, while at the same time allowing a message transmitted by electro-acoustic means to be heard, the messages being of the type in which the electrical signals carrying the said messages are mixed with the signals emitted by the said microphone, the said mixed signals then being sent to the said transducer (6) via the said amplifier (11), such that the said mixed signals also pass through the said filter (12).

7. Device for attenuating sounds of external origin arriving at the eardrum, of the type consisting of a passive means of soundproofing (1d, 1g) which surrounds a cavity (10) around each ear, and in addition, an electro-acoustic transducer (6) and a microphone (8) which are situated within the said cavity and are linked to one another by a feedback loop made up of a constant gain amplifier (11) and a filter (12) which constitute an active sound attenuation system, characterised by the transfer function C(w) of the said filter being a complex polynomial function and by the product of the constant gain (K) of the said amplifier (11) and the modulus |C(w)| of the transfer function of the said filter and the modulus |H(w)| of the open loop trabsfer function measured at the input (A) of the said transducer (6) and the output (B) of the said microphone (8) being much greater than one in the entire range of low acoustic frequencies which are to be attenuated and that the feedback loop be stable over all audible frequencies.

8. Device according to claim 7, characterised by the said filter (12) containing one or more analog filters of the pass-band or pass-band and low-pass type mounted in parallel and which produce a transfer function enabling the avoidance of instabilities in the zone where the modulus is greatest.

9. Device according to claim 7, characterised bu the said filter containing several analog filters of the low-pass, pass-band, and high-pass type, mounted in parallel and which have the same cut-off frequency and same coefficient of excess voltage.

10. Device according to claim 7, characterised by the gain (K) of the said amplifier (11) being positive and the transfer function C(w) of the said filter being determined such that the phase ($\phi$) of the said transfer function does not pass through the value of zero in the inband of the said filter.

11. Device according to claim 7, of the type in which each of the cavities contains a transverse partition (7) which divides it into two half-cavities, one forward and one rear, and which supports the said acoustic transducer and in which the said microphone (8) is situated in the said forward half-cavity, additionally characterised by containing a ring-shaped component (15) which is interposed between the said partition (8) and the ear pinna (4) and which limits an intermediate cavity (15a), the dimensions of the said ring-shaped component being calculated such that the ratio of the dimensions of the said intermediate cavity and those of the forward and rear half-cavities leads to an acoustic filtration in which the in-band corresponds to the range of frequencies to be attenuated.

12. Device according to claim 7, characterised by the said microphone (8) being placed in the auditory passage (3) and the said transducer (6) being a miniaturised transducer in which the covering of the front face forms a plug which is fitted into the entrance of the auditory passage (3) so that the said cavity is reduced to the volume of the auditory passage (3), the eardrum (16) and the said transducer (6) and that the open loop transfer function H(w) is very linear enabling electronic filtering and a good attenuation over a wide range of frequencies to be obtained.

13. Device according to claim 8, characterised by the said filter additionally containing one or more high-pass filters mounted in parallel with the said low-pass and pass-band filters.

**Ansprüche**

1. Verfahren zur Dämpfung von Lärm, der von außen kommt und zum Trommelfell gelangt, bei welchem Verfahren man an jedem Ohr ein mit diesem einen Hohlraum (10) begrenzendes passives Schalldämpungsmittel (1g, 1d) anbringt und man im Inneren des Hohlraums einen elektroakustischen Transducer (6) und ein Mikrofon (8) anordnet, welche über eine Gegenreaktionsschleife mit einen aktiven Schalldämpfer bildendem Verstärker (11) mit konstanter Verstärkung und Filter (12) miteinander verbunden sind, dadurch gekennzeichnet, daß die Übertragungsfunktion C(ω) des Filters (12) eine komplexe polynomische Funktion ist und daß man zuerst die Übertragungsfunktion in offener Schleife H(ω) der vom Transducer (6), dem Mikrofon (8) und dem vom passiven Schalldämpfungsmittel und dem Ohr begrenzten Hohlraum (10) gebildeten Gruppe mißt, und daß man danach die Koeffizienten der polynomischen Funktion C(ω) errechnet, damit das Produkt aus der konstanten Verstärkung (K) des Verstärkers (11) mit dem Modul der Übertragungsfunktion in offener Schleife /H(ω)/ und mit dem Modul der Übertragungsfunktion des Filters /c(ω)/ in dem Frequenzbereich, in dem das passive Schalldämpfungsmittel wenig wirksam ist, größer ist als 1, wobei aber die Stabilität des Gegenreaktionssystems erhalten bleibt.

2. Verfahren nach Anspruch 1, dadurch gekennzeichnet, daß man zur Messung der Übertragungsfunktion in offener Schleife H(ω) den Verstärker (11) und den Filter (12) abschaltet, man ein einem weißen Rauschen entsprechendes elektrisches Signal an den Eingang (A) des Transducers sendet, und man die Übertragungsfunktion mit einem Spektrumanalysator, der gleichzeitig das elektrische Signal und das vom Mikrofon (8) entsendete Signal empfängt, mißt.

3. Verfahren nach Anspruch 1, dadurch gekennzeichnet, daß man die Übertragungsfunktion in offener Schleife H(ω) durch Unterteilung des Hohlraums (10) mittels einer Trennwand (7) in zwei Halbräume, u.zw. einen von der Ohrmuschel (4), dem Gehörgang (3), dem Trommelfell (16) und der Trennwand (7) begrenzten vorderen Halbraum und einen vom passiven Schalldämpfungsmittel (1) und der Trennwand (7) begrenzten hinteren Halbraum, modifiziert, wobei die Trennwand (7) den Transducer (6) trägt, und man das Mikrofon (8) im vorderen Halbraum möglichst nahe bei der Emissionsfläche des Transducers (6) plaziert.

4. Verfahren nach Anspruch 3, dadurch gekennzeichnet, daß man das Volumen des Hohlraums (10) zwecks "Linearisierens" der Übertragungsfunktion in offener Schleife H(ω) so weit wie möglich reduziert.

5. Verfahren nach Anspruch 3, dadurch gekennzeichnet, daß man zwischen der Trennwand (7) und der Ohrmuschel (4) ein ringförmiges, einen Zwischenhohlraum (15a) begrenzendes Stück (15) einsetzt, und man die Form und die Dimensionen des ringförmigen Stücks (15) so berechnet, daß es eine akustische Filterung ausführt, die die Eingabe einer Tiefpaßfilter-oder Bandpaßfilterfunktion je nach dem zu dämpfenden Frequenzbereich in die Übertragungsfunktion in offener Schleife H(ω) gestattet.

6. Verfahren nach Anspruch 1 zur Dämpfung von Lärm, der von außen kommt, mit einem am Eingang der Ohren plazierten aktiven Dämpfungsmittel unter Ermöglichung des Verstehens einer auf elektroakustischem Weg übermittelten Nachricht, bei welchem man die die Nachrichten übermittelnden elektrischen Signale mit den vom Mikrofon entsendeten Signalen mischt und man die Mischsignale unter Leiten über den Verstärker (11) an den Transducer (6) anlegt, dadurch gekennzeichnet, daß die Mischsignale auch durch den Filter (12) gehen.

7. Vorrichtung zur Dämpfung von Lärm, der von außen kommt und zum Trommelfell gelangt, umfassend mit jedem Ohr einen Hohlraum (10) begrenzende passive Dämpfungsmittel (1d, 1g) und weiters einen elektroakustischen Transducer (6) und ein Mikrofon (8), die im Inneren des Hohlraums angeordnet und durch eine Gegenreaktionsschleife miteinander verbunden sind, welche einen Verstärker (11) mit konstanter Verstärkung und einen Filter (12) enthält, mit denen sie einen aktiven Geräuschdämpfer bilden, dadurch gekennzeichnet, daß die Übertragungsfunktion C(ω) des Filters eine komplexe polynomische Funktion ist, und daß das Produkt aus konstanter Verstärkung (K) des Verstärkers (11) mit dem Modul /c(ω)| der Übertragungsfunktion des Filters und mit dem Modul /H(ω)| der Übertragungsfunktion in offener Schleife, gemessen zwischen dem Eingang (A) des Transducers (6) und dem Ausgang (B) des Mikrofons (8), deutlich höher ist als 1 im gesamten Bereich der zu dämpfenden akustischen Niedrigfrequenzen und daß die Gegenreaktionsschleife für sämtliche hörbare Frequenzen stabil ist.

8. Vorrichtung nach Anspruch 7, dadurch gekennzeichnet, daß der Filter (12) einen oder mehrere analoge Filter des Bandpaß- oder des Bandpaß- und Tiefpaßtyps umfaßt, die parallelgeschaltet sind und eine Übertragungsfunktion ausführen, die die Vermeidung von Instabilitäten in der Zone, in der der Modul am stärksten ist, zuläßt.

9. Vorrichtung nach Anspruch 7, dadurch gekennzeichnet, daß der Filter mehrere analoge Filter des Tiefpaß-, Bandpaß-und Hochpaßtyps umfaßt, die parallelgeschaltet sind und dieselbe Schnittfrequenz und denselben Überspannungskoeffizienten haben.

10. Vorrichtung nach Anspruch 7, dadurch gekennzeichnet, daß die Verstärkung (K) des Verstärkers (11) positiv ist und die Übertragungsfunktion C($\omega$) des Filter derart festgelegt wird, daß die Phase ($\omega$) der Übertragungsfunktion nicht durch den Nullwert im Durchlaßband des Filters geht.

11. Vorrichtung nach Anspruch 7, bei welcher jeder der Hohlräume eine Querwand (7) aufweist, die ihn in einen vorderen und einen hinteren Halbraum teilt und den akustischen Transducer trägt, wobei das Mikrofon (8) im vorderen Halbraum angeordnet ist, dadurch gekennzeichnet, daß sie weiters ein ringförmiges Stück (15) umfaßt, welches zwischen der Trennwand (7) und der Ohrmuschel (4) eingesetzt ist und einen Zwischenhohlraum (15a) begrenzt, und daß die Dimensionen des ringförmigen Stücks (15) so berechnet sind, daß das Verhältnis zwischen den Dimensionen des Zwischenhohlraums und dem vorderen und hinteren Halbhohlraum zu einem akustischen Filtrat führt, dessen Durchlaßband dem zu dämpfenden Frequenzbereich entspricht.

12. Vorrichtung nach Anspruch 7, dadurch gekennzeichnet, daß das Mikrofon (8) im Gehörgang (3) plaziert ist und der Transducer (6) ein Minitransducer ist, dessen Verkleidung auf der Rückseite einen Stöpsel bildet, der so in den Eingang des Gehörganges (3) eingesetzt ist, daß der Hohlraum auf das vom Gehörgang (3), dem Trommelfell (16) und dem Transducer (6) begrenzte Volumen reduziert ist und die Übertragungsfunktion in offener Schleife H($\omega$) höchst linear ist und die Erzielung eines guten Dämpfungsniveaus in einem breiten Frequenzband durch elektronische Filterung gestattet.

13. Vorichtung nach Anspruch 8, dadurch gekennzeichnet, daß der Filter weiters einen oder mehrere Hochpaßfilter umfaßt, die mit den Tiefpaß- und Bandpaßfiltern parallelgeschaltet sind.

Fig.1

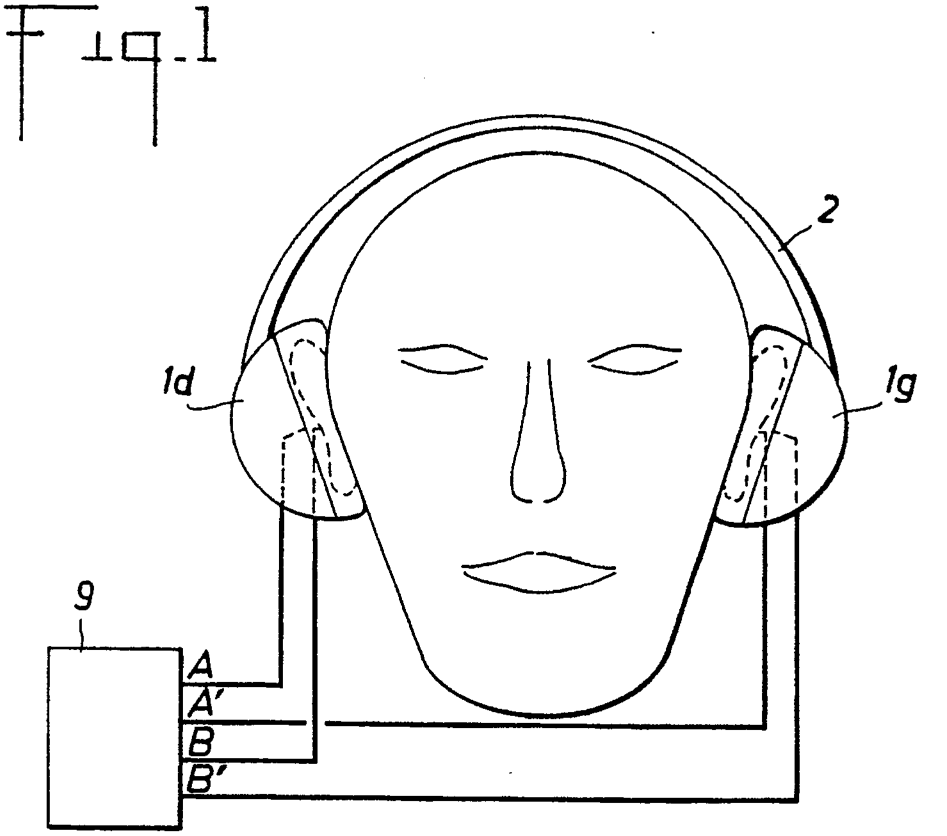


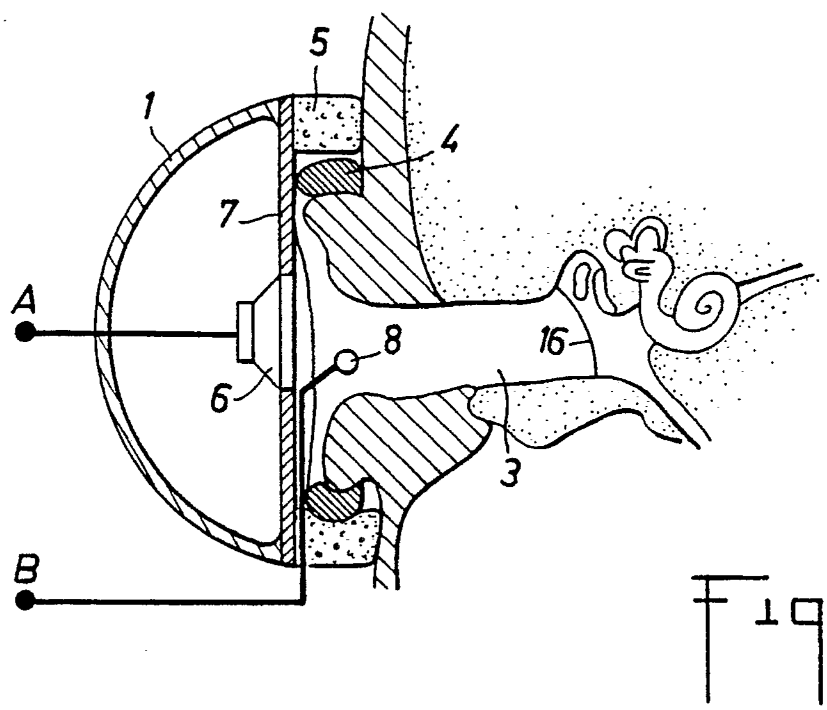


Fig.2

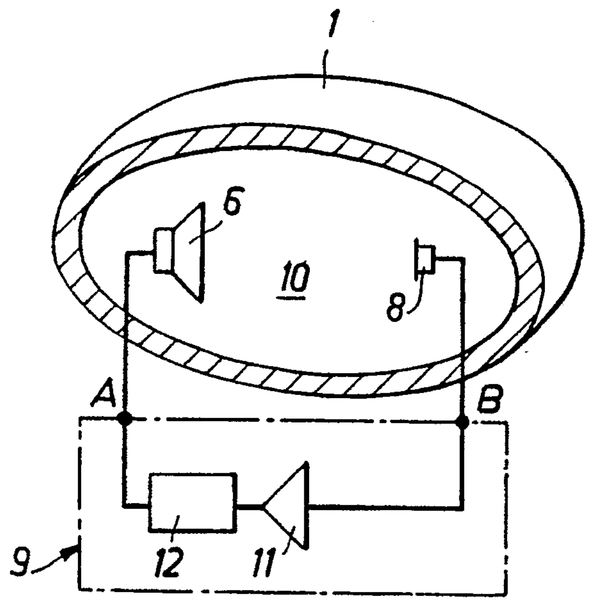


Fig. 3

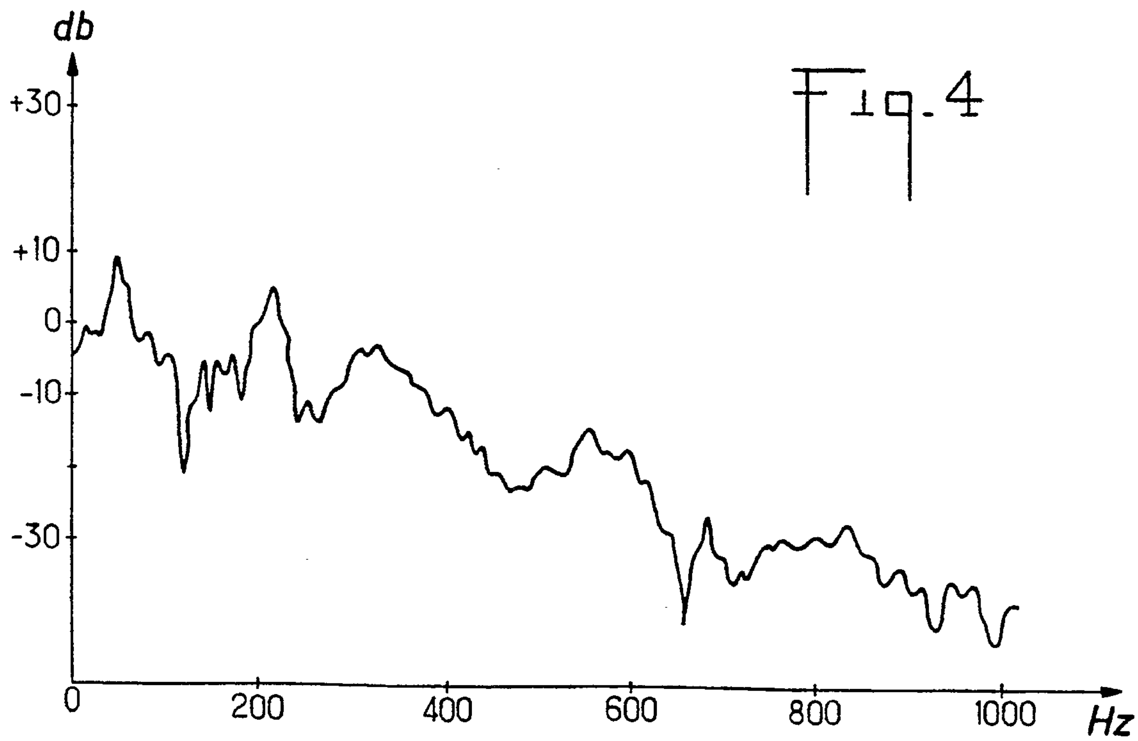


Fig. 4

Fig.6

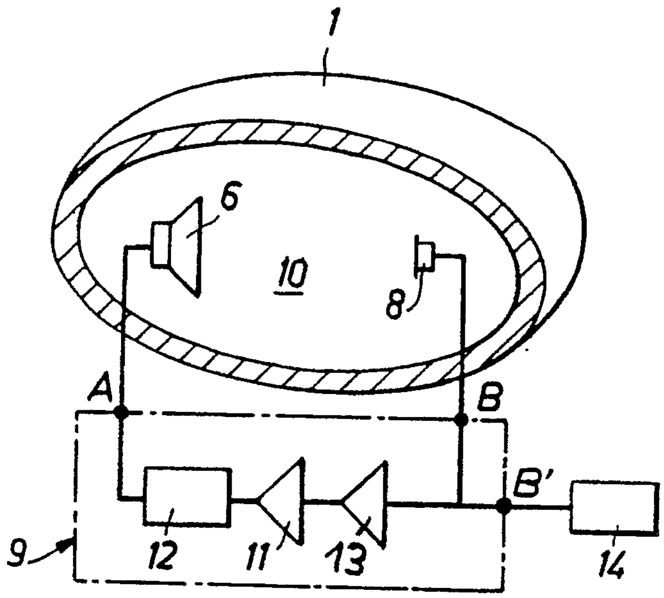
1
6
10
8
A
B
B'
9
12
11
13
14

Fig.5

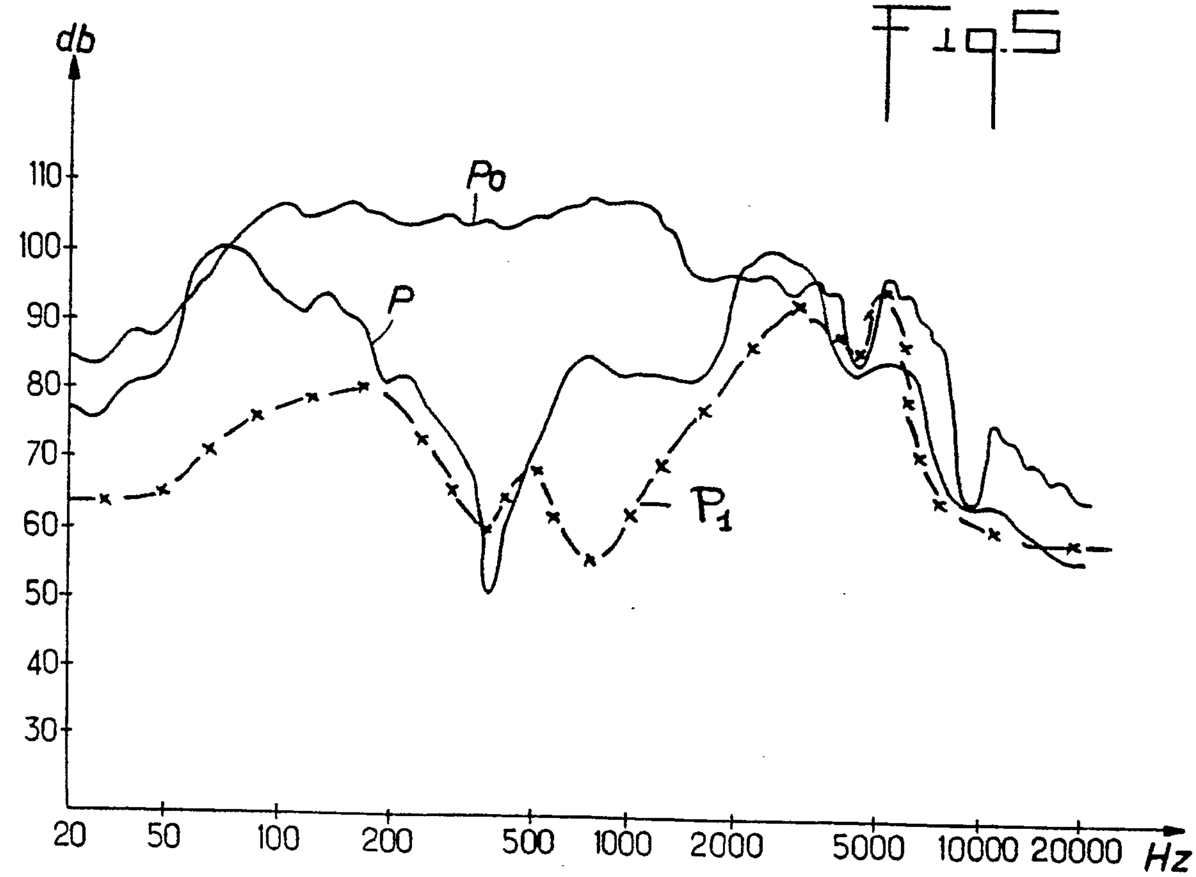
db
110
100
90
80
70
60
50
40
30
Po
P
P1
20
50
100
200
500
1000
2000
5000
10000
20000
Hz

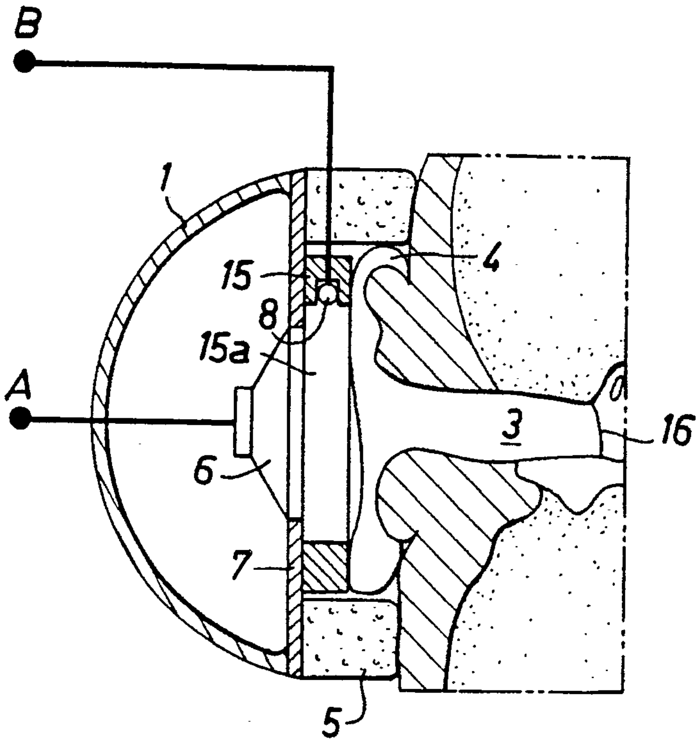


Fig. 7